# EUROPEAN PATENT APPLICATION

(11) **EP 4 717 155 A1**
(43) Date of publication of application: **01.04.2026**
(21) Application number: 25203163.8
(22) Date of filing: 18.09.2025
(51) Int. Cl.: A61B 5/00

(54) **SYSTEMS AND METHODS FOR MEDICAL DEVICE VISUALIZATION**

(30) Priority: 25.09.2024 US 202463698825 P
(71) Applicant: St. Jude Medical, Cardiology Division, Inc., St. Paul, MN 55117 (US)
(72) Inventor: AFONSO, Valtino X., Oakdale, 55128 (US); BIRD, Nathaniel, Minneapolis, 55414 (US); SCHWEITZER, Jeffrey, St. Paul, 55108 (US)
(74) Representative: Mathys & Squire

(57) **Abstract**

A visualization system for a medical procedure. The system includes a medical device, a localization system, a signal processing system, and a display. The medical device includes one or more position sensors. The localization system is configured to generate an magnetic field and/or an impedance field to track the one or more position sensors. The signal processing system is configured to receive input location signals from the one or more position sensors. The signal processing system includes low pass filter to attenuate oscillation in the input location signals from cardiac motion and a low pass filter controller to generate a reset output communicated to the low pass filter to reset the low pass filter. The display provides real-time visualization of the medical device.

## Description

### TECHNICAL FIELD

The subject matter disclosed herein relates to mapping and visualization systems and, in particular, to electrophysiology visualization systems for medical procedures.

### BACKGROUND

Mapping and visualization systems are used for various electrophysiological procedures. For example, an electrophysiology catheter including a plurality of sensors or electrodes can be manipulated within or around the heart to measure electrophysiology data at various locations of the heart. The electrophysiology data and location data (e.g., position and orientation data) can be utilized to construct a visualized model of the heart and/or a visualized model of the electrophysiology catheter.

The location data can be distorted by cardiac motion, i.e., the motion of the cardiac tissue as the heart beats can move the sensors or electrodes on the electrophysiology catheter relative to a fixed coordinate system (e.g., a magnetic field-based and/or an impedance-based coordinate system). A low pass filter can be used to attenuate the high frequency oscillation in the location data. The attenuation of the cardiac motion is beneficial for a visualization system, as the visualized model of the heart and catheter will remain stable. However, the low pass filter introduces latency or lag in the visualized model of the catheter, thereby decreasing the responsiveness of the visualization system. There exists a need for an improved visualization system that provides high responsiveness while attenuating cardiac motion.

### SUMMARY

In some aspects, the techniques described herein relate to a visualization system for a medical procedure, the system including: a medical device including one or more position sensors; a localization system to generate location data based on input received from the one or more position sensors; a signal processing system configured to receive the location data from the localization system, the signal processing system including: a low pass filter configured to generate filtered location data in response to the location data provided by the localization system, and a low pass filter controller configured to monitor the location data provided as an input to the low pass filter and the filtered location data to generate a reset output communicated to the low pass filter to reset the low pass filter; and a display providing real-time visualization of the medical device.

In some aspects, the techniques described herein relate to a visualization system, wherein the reset of the low pass filter includes increasing a sample rate of the input location signals input into the low pass filter.

In some aspects, the techniques described herein relate to a visualization system, wherein the reset of the low pass filter includes inputing a location estimate signal into the low pass filter.

In some aspects, the techniques described herein relate to a visualization system, wherein the reset output includes a reset metric, wherein one or more input parameters of the low pass filter are reconfigured based on the reset metric.

In some aspects, the techniques described herein relate to a visualization system, wherein the low pass filter controller computes the location estimate signal of the one or more position sensors on the medical device, wherein the location estimate signal is computed by a moving statistical filter over a time interval.

In some aspects, the techniques described herein relate to a visualization system, wherein the reset metric is computed by determining a difference between the location estimate signal of the one or more position sensors on the medical device and the input location signals from the one or more position sensors.

In some aspects, the techniques described herein relate to a visualization system, wherein the low pass filter controller determines whether a reset event has occurred by comparing the difference to a threshold, wherein if the difference is greater than the threshold the reset event has occurred and the reset output is generated.

In some aspects, the techniques described herein relate to a method of displaying a visualized medical device during a medical procedure, the method including: tracking one or more position sensors on a medical device with a positioning system; inputting location data of the one or more position sensors into a low pass filter to attenuate oscillation of the location data from cardiac motion; inputting the location data of the one or more position sensors into a low pass filter controller to determine whether a reset event occurred; outputting a reset signal from the low pass filter controller to the low pass filter; resetting the low pass filter; and outputting filtered location data to a display.

In some aspects, the techniques described herein relate to a method, wherein resetting the low pass filter reduces latency of the filtered location data.

In some aspects, the techniques described herein relate to a method, wherein resetting the low pass filter includes modifying one or more input parameters of the low pass filter, wherein the one or more input parameters include a sample rate of the low pass filter.

In some aspects, the techniques described herein relate to a method, wherein resetting the low pass filter includes inputting the location data from a previous time interval into the low pass filter.

In some aspects, the techniques described herein relate to a method, further including: computing a location estimate of the one or more position sensors on the medical device with a moving statistical filter over a time interval computing a reset metric by determining a variance between the location estimate of the one or more position sensors on the medical device and the location data from the one or more position sensors, and comparing the variance to a threshold to determine whether the reset event has occurred.

In some aspects, the techniques described herein relate to a method, wherein resetting the low pass filter includes inputting the location estimate of the one or more position sensors into the low pass filter.

In some aspects, the techniques described herein relate to a method of attenuating cardiac motion from medical device position data of a medical device, the method including: inputting location signals from one or more position sensors disposed on the medical device into a low pass filter to generate low pass filtered location data; inputting the location signals from the one or more position sensors into a low pass filter controller; determining whether motion of the one or more position sensors is attributable to manipulation of the medical device by a user; generating a reset output with the low pass filter controller when motion of the one or more position sensors is attributable to manipulation of the medical device by the user; and resetting the low pass filter.

In some aspects, the techniques described herein relate to a method, wherein resetting the low pass filter includes modifying one or more input parameters of the low pass filter, wherein the one or more input parameters include a sample rate of the low pass filter.

In some aspects, the techniques described herein relate to a method, wherein the one or more input parameters include a cutoff frequency, a normalized passband frequency, a filter order, and/or a filter type.

In some aspects, the techniques described herein relate to a method, wherein resetting the low pass filter includes inputting the location signals from a previous time interval into the low pass filter.

In some aspects, the techniques described herein relate to a method, wherein determining whether motion of the one or more position sensors is attributable to manipulation of the medical device by the user includes: computing an estimated cardiac motion parameter, computing a difference between the location signals from one or more position sensors and the estimated cardiac motion parameter, and comparing the difference to a threshold value.

In some aspects, the techniques described herein relate to a method, wherein the estimated cardiac motion parameter includes one or more of a maximum distance displacement of the location signals, a slew rate of the location signals, and a slew rate of the low pass filtered location data.

In some aspects, the techniques described herein relate to a method, wherein resetting the low pass filter includes inputting a location estimate of the one or more position sensors into the low pass filter, wherein the location estimate is computed via a moving statistical filter within a time interval of previous data.

### BRIEF DESCRIPTION OF THE DRAWINGS

**FIG. 1** is a is a diagrammatic view of a medical system including a medical positioning system, according to some embodiments.
**FIG. 2A** is a diagrammatic view of a medical system including a catheter positioned in the heart of a patient, according to some embodiment.
**FIG. 2B** is a side view of a distal end of a catheter of a medical system, according to some embodiments.
**FIG. 3** is a diagrammatic view of a signal processing system for a medical system, according to some embodiments.
**FIG. 4** is a diagrammatic view of a signal processing system for a medical system, according to some embodiments.
**FIG. 5** is an exemplary plot of catheter location versus time, according to some embodiments.
**FIG. 6** is an exemplary plot of catheter location versus time, according to some embodiments.
**FIG. 7** is a flow chart of a method of displaying a visualized medical device during a medical procedure, according to some embodiments.

### DETAILED DESCRIPTION

The present disclosure describes a signal processing system or method for a medical device visualization system configured to reduce latency of the filtered location signals generated by a low-pass (LP) filter. The signal processing system includes an LP filter controller configured to selectively reset the LP filter if motion indicative of medical device manipulation (e.g., deliberate motion of the catheter) occurs. The reset of the LP filter includes modifying one or more input parameters of the LP filter, including for example, a sample rate of the LP filter, and/or modifying the signals input into the LP filter. The reset of the LP filter improves responsiveness (i.e., reduces latency) of the filtered location data signals.

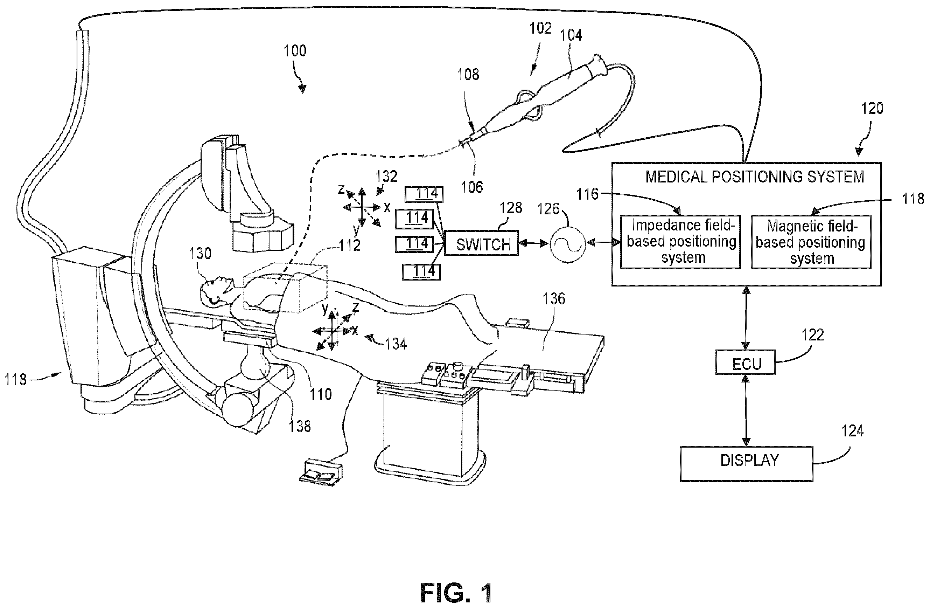

**FIG. 1** is a diagrammatic view of a medical system 100 including a medical positioning system 120, according to some embodiments. The medical system 100 includes a medical device 102 having a handle 104, a shaft 106, and a proximal end 108. In some embodiments, the medical positioning system 120 includes an impedance-based positioning system 116 and/or a magnetic field-based positioning system 118. In other embodiments, various other types of medical positioning systems may be utilized, either alone or in combination with the medication positioning systems shown in **FIG. 1****.** The magnetic field-based positioning system 118 includes a magnetic field generator 110 configured to generate a magnetic field that is sensed by magnetic sensors located on the medical device 102 and utilized to generate magnetic-based location data. The impedance-based positioning system 116 includes surface patch electrodes 114, a signal generator 126, and a switch 128, wherein excitation signals provided to the patch electrodes 114 create an impedance field (or electric field) throughout the patient's body. Electrodes located on the medical device 102 sense the impedance field and generate in response a sensor signal utilized to generate impedance-based location data. In some embodiments, the medical system further includes an electronic control unit (ECU) 122 and a display 124. In some embodiments, ECU 122 receives location data from the medical positioning system and displays location data to a physician/technician via the display 124. In some embodiments, ECU 122 may provide additional processing of the location data as described in more detail below to improve the location data presented to the user via the display. For example, this may include filtering the location data to remove oscillations caused by the beating of the patient's heart. In some embodiments, these functions (e.g., filtering) are performed by the ECU 122. In other embodiments, these functions may be performed by the medical positioning system 120.

The medical device 102 can include an elongated medical device such as, for example, a catheter or a sheath. For purposes of illustration and clarity, the description below will be limited to an embodiment wherein the medical device 102 comprises a catheter (e.g., catheter 102). It will be appreciated, however, that the present disclosure is not meant to be limited to such an embodiment, but rather in other example embodiments, the medical device may comprise other medical devices, such as, for example and without limitation, sheaths, guidewires and the like. In yet further embodiments, the catheter 102 may be any medical device wherein real-time location-based data may be advantageous to a procedure in which it is used.

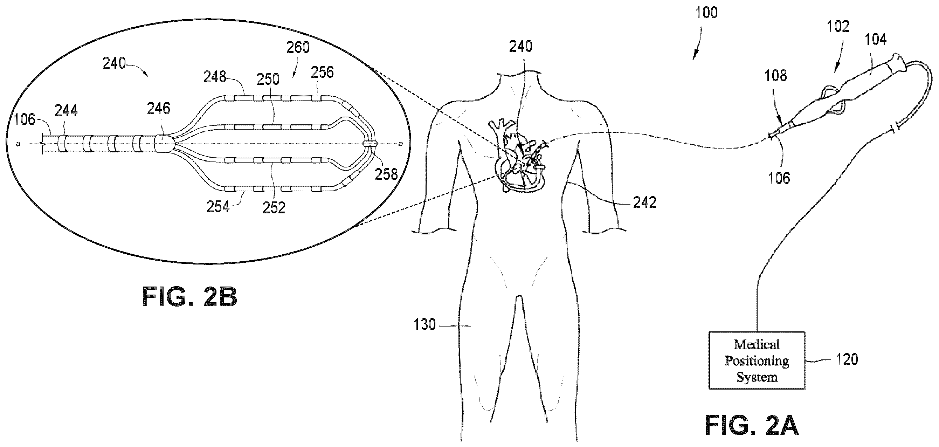

A distal tip (e.g., distal end 240 shown in **FIG. 2B****)** of catheter 102 can be inserted into the body 130, and more particularly, into the heart. The catheter 102 may include the handle 104, the shaft 106 having the proximal end 108 and a distal end including one or more sensors mounted in or on the shaft 106 of the catheter 102. The sensors may include a catheter electrode and/or a magnetic sensor, and monitoring by the one or more sensors is communicated via the shaft 106 to the handle 104 and is received by the medical positioning system 120. In some embodiments, both the impedance-based positioning system 116 and the magnetic field-based positioning system 118 generate corresponding position estimates. In some embodiments, ECU 122 receives both impedance based position estimates and magnetic based position estimates. The catheter 102 may further include other conventional components such as, for example and without limitation, a temperature sensor, additional sensors or electrodes, ablation elements (e.g., ablation tip electrodes for delivering RF ablative energy, high intensity focused ultrasound ablation elements, etc.), and corresponding conductors or leads. The shaft 106 can be an elongated, tubular, flexible member for movement within the body 130. The shaft 106 supports, for example and without limitation, sensors and/or electrodes mounted thereon, such as, for example, sensors, associated conductors, and possibly additional electronics used for signal processing and conditioning. The shaft 106 may also permit transport, delivery, and/or removal of fluids (including irrigation fluids, cryogenic ablation fluids, and bodily fluids), medicines, and/or surgical tools or instruments. The shaft 106 may be made from conventional materials such as polyurethane, and define one or more lumens configured to house and/or transport electrical conductors, fluids, or surgical tools. The shaft 106 may be introduced into a blood vessel or other structure within the body 130 through a conventional introducer. The shaft 106 may then be steered or guided through the body 130 to a desired location, such as the heart, using means well known in the art.

Some embodiments of the medical positioning system 120 will now be described. The medical positioning system 120 can be provided for determining a position and/or orientation of the catheter 102, and thus, the position and/or orientation of a distal portion of the catheter 102. In some embodiments, the medical positioning system 120 comprises a magnetic field-based system such as, for example, the MediGuide^{™} system from MediGuide Ltd. (now owned by St. Jude Medical, Inc.), and as generally shown with reference to one or more of U.S. Pat. Nos. 6,233,476; 7,197,354; and 7,386,339, the entire disclosures of which are incorporated herein by reference. In some embodiments, the medical positioning system 120 comprises an impedance-based system such as, for example, the ENSITE NAVX system sold by St. Jude Medical, Inc. of St. Paul, Minn., and described in, for example, U.S. Pat. No. 7,263,397 titled "Method and Apparatus for Catheter Navigation and Location Mapping in the Heart," the entire disclosure of which is hereby incorporated by reference as though fully set forth herein.

The display 124 is provided to convey information to a physician to assist in diagnosis and treatment. The display 124 may comprise one or more conventional computer monitors or other display devices. The display 124 may present a graphical user interface (GUI) to the physician. The GUI may include a variety of information including, for example, an image of the geometry of the tissue/heart, electrophysiology data associated with the heart, graphs illustrating voltage levels over time for various sensors, and visualized models of the catheter 102 and other medical devices relative to the body 130.

**FIGS. 2A-B** illustrate diagrammatic view of a heart 242 with the catheter 102 inserted therein, according to some embodiments. In the embodiment shown in **FIG. 2A****,** the shaft 106 of the catheter 102 extends into a left ventricle of the heart 242, however, it is contemplated that the shaft 106 of the catheter 102 may be positioned within or around other chambers or vessels of the heart 242. **FIG. 2B** is a magnified view of the distal end 240 of the catheter 102 according to some embodiments. The distal end of the catheter 102 may utilize a variety of geometries and configurations, and the present disclosure is not limited to the configuration shown in **FIG. 2B****.**

As shown in **FIG. 2B****,** the distal end 240 of the catheter 102 includes one or more shaft electrodes 244, a connector 246, and a flexible head 260. The flexible head 260 includes a first arm 248, a second arm 250, a third arm 252, and a fourth arm 254, each including one or more electrodes 256 and secured together via a distal connector 258, according to some embodiments.

The one or more shaft electrodes 244 disposed along the length of the shaft 106 can be used for diagnostic, therapeutic, and/or mapping procedures, in an example. For instance, the impedance-based positioning system 116 can be configured to track or measure the position of the one or more shaft electrodes 244 in the impedance-based coordinate system 132. In some embodiments, the catheter 102 and/or the distal end 240 includes one or more magnetic sensors (e.g., a coil wrapped around a magnetically permeable core) configured to sense a generated magnetic field. The magnetic field-based positioning system 118 can be configured to track or measure the position of the one or more magnetic sensors in the magnetic-based coordinate system 134.

The flexible head 260 of the catheter 102 can be adapted to conform to tissue (e.g., cardiac tissue). For example, when the flexible head 260 contacts tissue, the flexible head 260 can deflect, allowing the flexible framework to conform to the tissue. In some embodiments, the arms (or the understructure of the arms) comprising the paddle structure (or multi-arm, electrode-carrying, flexible framework) at the distal end 240 of the catheter 102 are constructed from a flexible or spring-like material such as Nitinol and/or a flexible substrate, as discussed herein.

In some embodiments, the one or more electrodes 256 of the flexible head 260 are configured to (1) define regional propagation maps of particularly sized areas (e.g., one centimeter square areas) within the atrial walls of the heart; (2) identify complex fractionated atrial electrograms for ablation; (3) identify localized, focal potentials between the one or more electrodes 256 for higher electrogram resolution; (4) to precisely target areas for ablation; and/or (5) to configured to track or measure the position of the one or more electrodes 256 in the impedance-based coordinate system 132. These mapping catheters and ablation catheters are constructed to conform to, and remain in contact with, cardiac tissue despite potentially erratic cardiac motion. Such enhanced stability of the catheter on a heart wall during cardiac motion provides more accurate mapping and ablation due to sustained tissue-electrode contact. Additionally, the catheters described herein may be useful for epicardial and/or endocardial use. For example, the planar array embodiments depicted herein may be used in an epicardial procedure where the planar array of microelectrodes is positioned between the myocardial surface and the pericardium. Alternatively, the planar array embodiments may be used in an endocardial procedure to quickly sweep and/or analyze the inner surfaces of the myocardium and quickly create high-density maps of the heart tissue electrical properties.

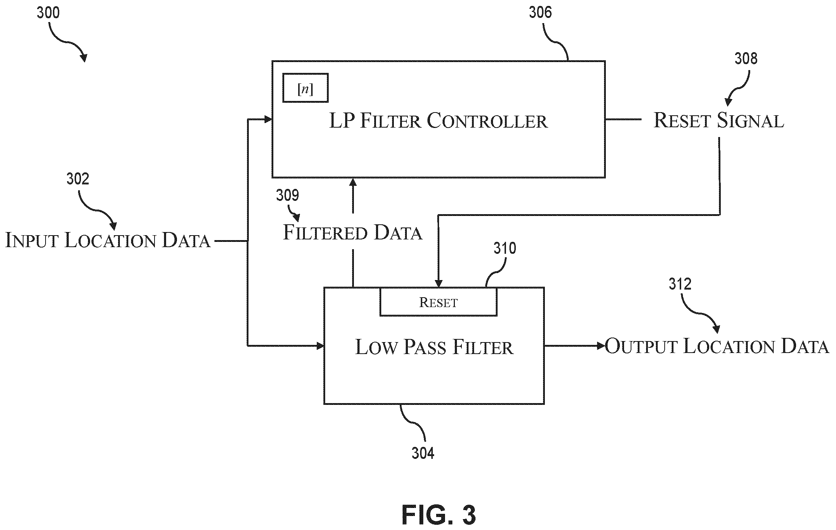

**FIG. 3** is a diagrammatic view of a signal processing system 300 for the medical system 100, according to some embodiments. In some embodiments, the signal processing system is implemented as part of the ECU 122 (shown in **FIG. 1****)** and operates on location data provided by the medical positioning system 120. In other embodiments, the signal processing system 300 may be implemented as part of the medical positioning system 120. The signal processing system 300 receives as an input location data 302, which is provided to a low pass filter 304. In general, the low pass filter operates to remove oscillations from the input location data 302, typically due to the beating of the heart, to generate filtered location data 312. In addition, the signal processing system 300 includes the LP filter controller 606 (reset module) configured to generate a reset signal provided to the low pass filter 304. The low pass filter 304 processes the input location data 302 and/or the reset signal 308 to generate output location data 312, according to some embodiments.

The input location data 302 include raw impedance-based position data of the one or more shaft electrodes 244, the one or more electrodes 256, and/or other electrodes or impedance sensors disposed on the catheter 102, according to some embodiments. The input location signals 302 include raw magnetic-based position data of the one or more magnetic sensors disposed on or within the catheter 102, according to some embodiments. The term "raw" refers to unprocessed or unfiltered position data. In some embodiments, the raw impedance-based position data and/or the raw magnetic-based position data is converted to position coordinates (e.g., in the impedance-based coordinate system 132 and/or the magnetic-based coordinate system 134) prior to being input into the signal processing system 300.

Low pass filter 304 may be implemented in a number of different ways. For example, in some embodiments, the low pass filter 304 includes an infinite impulse response (IIR) filter based on a cutoff frequency which attenuates frequencies above (or below) the cutoff frequency. In some embodiments, the low pass filter 304 includes a plurality of IIR filters operating in sequence, each providing a different low pass characteristic which results in a composite low pass filter. In some embodiments, the low pass filter 304 includes a proportion-integral-derivative (PID) controller to act as a filter, regulating how quickly the output location data 312 reacts to changes in the input location data 302, thereby removing undesirable oscillations. In some embodiments, the low pass filter 304 includes an adaptive filter (e.g., a Kaman filter) which has a priori data or can track and learn a model to attenuate the cardiac motion oscillation. In some embodiments, the low pass filter 304 includes a singular value decomposition (SVD) algorithm to learn and remove the cardiac motion oscillation. For instance, the SVD algorithm includes any and/or all features of the respiration compensation methods described in commonly-owned, U.S. Patent No. 9,113,807 entitled "Dynamic adaptive respiration compensation with automatic gain control," the contents of which are incorporated by reference in its entirety. In some embodiments, the low pass filter 304 includes a slew rate type processor which attenuates high frequency-type cardiac motion. In some embodiments, the low pass filter 304 includes a finite impulse response (FIR) filter based on a cutoff frequency which attenuates frequencies above (or below) the cutoff frequency. The low pass filter 304 includes any combination of the above filters and features described above, as well as any other low pass filters or low pass filter features commonly known in the art, according to some embodiments.

In general, the low pass filter 304 attenuates high frequency oscillation in the input location data 302, i.e., the oscillation of the location data due to cardiac motion (heartbeat) acting on the catheter 102. The low pass filter 304 introduces latency (i.e., a time delay on the real-time position) on the location data, and thereby negatively impacts the responsiveness of the medical positioning system 120 as the catheter 102 is manipulated by the physician. The signal processing system 300 is configured to reduce the latency of the medical positioning system 120 introduce by the low pass filter 304.

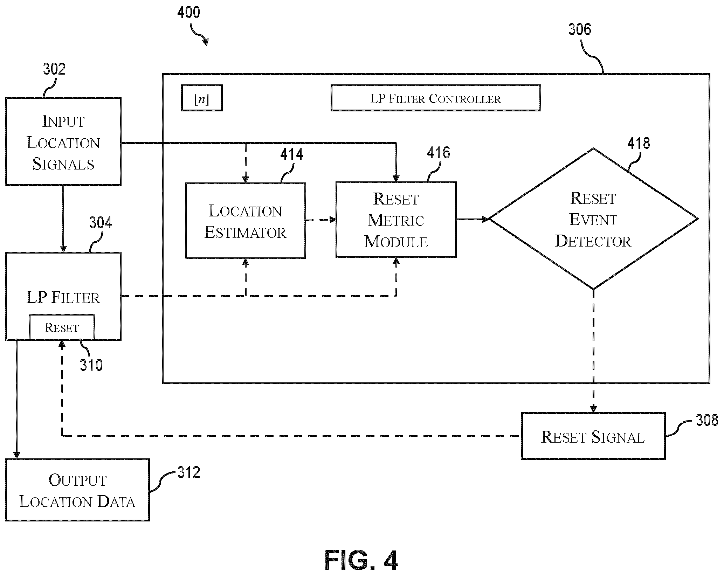

The LP filter controller 306 receives the input location signals 302 and/or filtered data 309 from the low pass filter 304 over a sample index [n] (i.e., a moving time period). The LP filter controller 306 determines whether the low pass filter 304 should be reset based on the received signals. For instance, the LP filter controller 306 analyzes the input location signals 302 to determine whether the catheter has been moved by the physician (i.e. user deliberate motion), and if so, generates the reset signal 308. The LP filter controller 306 and embodiments thereof is described further in **FIG. 4****.**

The LP filter controller 306 generates the reset signal 308 based on the input location signals 302 and/or the filtered data 309 from the low pass filter 304. The reset signal 308 is communicated to the low pass filter 304, and in some embodiments, the reset signal 308 is input into the reset function 310 of the low pass filter 304. The low pass filter 304 generates output location data 312 based on the input location signals 302 and/or the reset signal 308 input into the low pass filter 304.

The output location data 312 includes position data of the one or more shaft electrodes 244, the one or more electrodes 256, the one or more magnetic sensors, etc., in the impedance-based coordinate system 132 or the magnetic-based coordinate system 134. In some embodiments, the position of each individual sensor/electrode is filtered separately in each coordinate of the respective coordinate system. For instance, the 3D input location signals from the proximal-most shaft electrode (e.g., the shaft electrode 244 shown in **FIG. 2B** tracked in the impedance-based coordinate system 132) can be separated into respective (x, y, z) components, with each component (x-component, y-component, z-component) filtered through the signal processing system 300. In some embodiments, the position of each sensor/electrode is filtered together, e.g., the oscillation in position data of a first electrode/sensor is used to predict the oscillation of a second electrode/sensor.

**FIG. 4** is a diagrammatic view of a signal processing system 400 for a medical system, according to some embodiments. The signal processing system 400 includes the LP filter controller 306. In some embodiments, the LP filter controller 306 includes a location estimator 414, a reset metric module 416, and/or a reset event detector 418.

The location estimator 414 of the LP filter controller 306 computes a robust, noise-free estimate of the position of a sensor or electrode of the catheter 102. In some embodiments, the location estimator 414 of the LP filter controller 306 removes oscillation from the input signals 302 (similar to the LP filter 304), however, the LP filter controller operates with less latency that the LP filter 304. For instance, the sampling time interval of the location estimator 414 is less than the sampling time interval of the LP filter 304 and/or the sampling rate of the location estimator 414 is greater than the sampling rate of the LP filter 304.

In some embodiments, the location estimator 414 utilizes a low pass filter approach to eliminate rapid changes in catheter location which are not attributable to catheter manipulation (i.e., movement of the catheter 102 via the physician). The input location signals 302 is input into the location estimator 414, according to some embodiments. In some embodiments, an output from the low pass filter 304 (e.g., the filtered data 309) is input into the location estimate 414 to determine the estimated position of the catheter 102. For instance, depending on the type of low pass filter 304 used (e.g., PID filter, adaptive filter, SVD algorithm filter, etc.), the low pass filter 304 can be used to learn and remove the oscillation from cardiac motion and provide an estimate of catheter position.

In some embodiments, the location estimator 414 includes one or more statistical filters, e.g., a statistical average of position over N samples or an exponential moving average filter using a short time interval, to determine the estimated position of the catheter 102. The statistical filters removed oscillation from cardiac motion and provide a rapidly updating estimate of catheter position. For instance, a moving average filter using a short interval of data (e.g., between 50 milliseconds and 300 milliseconds) updates continuously and removes oscillation in position data.

The reset metric module 416 of the LP filter controller 306 determines whether the catheter 102 has been manipulated by the physician to a new position within the heart, and thus, whether the low pass filter 304 should be reset. In some embodiments, the reset metric module 416 inputs the input location signals 302 and location estimate signals from the location estimator 414 and computes the difference between the input location signal 302 and the location estimate signals of a given sensor/electrode. In some embodiments, the reset metric module 416 inputs an estimated location from the location estimator 414 and the output of the low pass filter 304 and computes the difference between the estimated location from the location estimator 414 and the low pass filtered data of a given sensor/electrode.

In some embodiments, the reset metric module 416 computes a rate of change of catheter location. For instance, the reset metric module 416 inputs the input location signals 302 and calculates a slew rate (i.e., a maximum rate of change) of the input location signals 302 and/or inputs location estimate signals from the location estimator 414 and calculates a slew rate of the location estimate signals.

The reset metric module 416 communicates with the reset event detector 418 to determine whether the low pass filter 304 should be reset. The reset event detector 418 includes comparing the output of reset metric module 416 to a threshold value to determine whether catheter manipulation has occurred. In some embodiments, the reset event detector 418 includes comparing the output of the reset metric module 416 to a predetermined threshold value. For instance, in embodiments wherein the output of the reset metric module 416 is the difference between the input location signal 302 and the location estimate from the location estimator 414 of a given sensor/electrode, the reset event detector 418 determines whether the calculated difference exceeds a predetermined threshold indicative of catheter manipulation by the physician.

In some embodiments, the reset event detector 418 computes an adaptive threshold value. For instance, the threshold value includes an adaptively estimated cardiac motion, according to some embodiments. The adaptively estimated cardiac motion is based on the input location signals 302 from a prior time interval including a cardiac cycle (i.e., at least one heartbeat). A maximum displacement of the location data is calculated during the prior time interval, according to some embodiments. A maximum rate of change of catheter position is calculated during the prior time interval, according to some embodiments. The maximum displacement value and/or the maximum rate of change value of the prior time interval is incorporated in the adaptive threshold value, according to some embodiments. Thus, any displacement of the catheter 102 which exceeds the maximum displacement and/or the maximum rate of change is indicative of catheter manipulation, and therefore, a reset of the low pass filter 304 is beneficial to reduce the latency of catheter motion. The reset event detector 418 generates the reset signal 308 in response to detected catheter manipulation. The reset signal 308 is a signal communicated to the reset function 310 of the low pass filter 304 to reset the low pass filter 304.

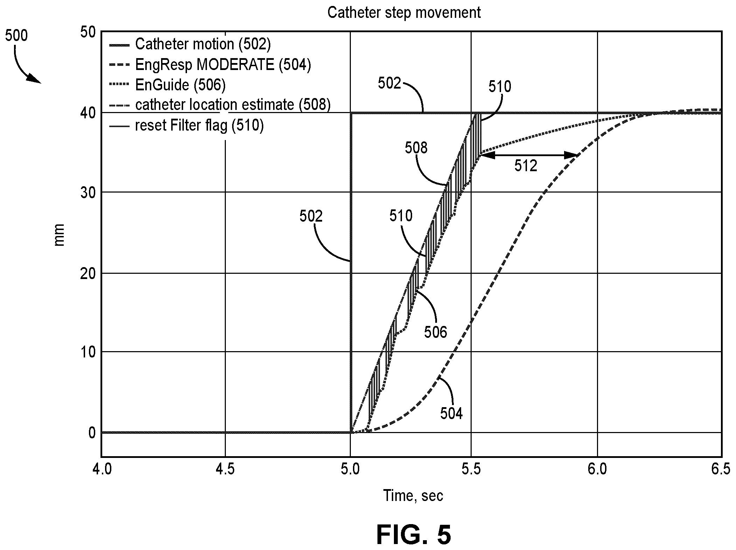

In some embodiments, resetting of the low pass filter 304 includes reconfiguring the inputs, or input settings, of the low pass filter 304. For instance, the location estimator 414, which provides a rapidly updating, noise free estimate of catheter position, is input into the low pass filter 304. Therefore, both the input location signals 302 and the location estimate from the location estimator 414 are input into the low pass filter 304 (as opposed to solely the input location signals 302), which drives the low pass filter 304 to output location data which rapidly approaches the location estimate. In some embodiments, resetting the low pass filter 304 includes increasing the input rate (or sample rate) of data which is input into the low pass filter 304. For instance, the sample rate of data input into the low pass filter 304 from the input location signals 302 can be increased from 2,000 samples per second (N) to 100,000 samples per second. Increasing the sample rate of data input into the low pass filter 304 increases the rate of change of the output (i.e., the latency of the catheter position data is decreased). **FIGS. 5-6** illustrate the effect that resetting the low pass filter 304 has on response time of the signal processing system 400.

**FIG. 5** is an exemplary plot 500 of catheter location versus time in a single coordinate (e.g., the x-direction), according to some embodiments. Real catheter position 502 is shown, as for example, the physician moves the catheter 40mm in the x-direction in a short period of time at the 5-second mark.

Low pass filter location data 504 comprises the output of the low pass filter 304 with input location signals 302 input into the low pass filter 304 and without input or communication from the LP filter controller 306. The low pass filter location data 504 lags behind the real catheter position 502. For instance, the low pass filter location data 504 approaches the real catheter position 502 after the 6.0 second mark-and therefore, the latency introduced by the low pass filter (e.g., the low pass filter 304) is greater than 1.0 seconds, according to some embodiments. The high latency period is detrimental for real-time visualization of the catheter relative to the heart or a model of the heart, as for example, the real-time visualization will take greater than 1.0 seconds to update the true, real-world location of the catheter.

Signal processing location data 506 from the signal processing system 300 and/or the signal processing system 400 is shown on the plot 500. The signal processing location data 506 comprises the output location data 312 from the signal processing system 300 and/or the signal processing system 400, with the LP filter controller 306 resetting the low pass filter 304 at reset indicators 510. Catheter location estimate data 508 is calculated via the location estimate 414 of the LP filter controller 306, according to some embodiments.

The reset indicators 510 illustrate where the LP filter controller 306 generated the reset signal 308 to trigger a reset of the low pass filter 304. In some embodiments, the reset metric 416 includes the distance between the catheter location estimate data 508 and the signal processing location data 506, i.e., when the difference in the estimated catheter location and the processed catheter location exceeds a threshold amount, the reset signal 308 is triggered to reset the low pass filter 304. The reset of the low pass filter 304 includes inputting the signal processing location data 506, and in some case, the estimated location data, into the low pass filter 304, according to some embodiments. The reset of the low pass filter 304 includes increasing a sample rate of data input into the low pass filter 304, according to some embodiments.

The signal processing location data 506 has better responsiveness than the low pass filter location data 504. For instance, a latency differential 512 is shown in FIG. 5 between the signal processing location data 506 and the low pass filter location data 504. The signal processing location data 506 is roughly 0.4 seconds ahead of (i.e., more responsive than) the low pass filter location data 504, according to some embodiments.

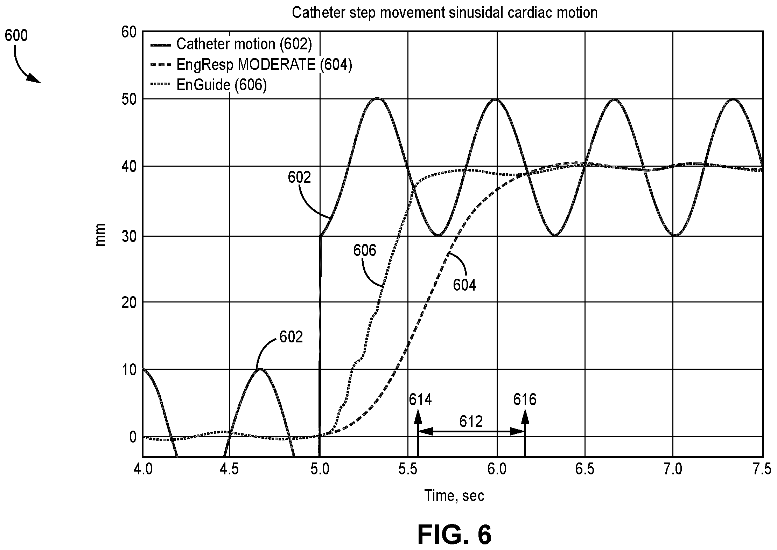

**FIG.** 6 is an exemplary plot 600 of catheter location versus time in a single coordinate (e.g., x-coordinate), with the catheter exposed to cardiac motion, according to some embodiments. Real catheter location 602 is shown with an oscillating motion from the repeated cardiac motion. At *t*=5.0 seconds, the catheter is moved 40mm in the x-direction.

Low pass filter location data 604 includes the output of the low pass filter 304 with input location signals 302 input into the low pass filter 304 and without input or communication from the LP filter controller 306. The low pass filter location data 604 attenuates the oscillation of catheter motion due to cardiac motion, however, the low pass filter location data 604 lags behind the real-time, real world catheter location (the real catheter location 602). For instance, dashed line 616 indicates where the low pass filter location data 604 catches up to the real catheter location 602. The dashed line 616 is positioned after *t*=6.0 seconds, indicating that the latency of the low pass filter location data 604 is greater than 1.0 seconds.

Signal processing location data 606 from the signal processing system 300 and/or the signal processing system 400 is shown on the plot 600. The signal processing location data 606 comprises the output location data 312 from the signal processing system 300 and/or the signal processing system 400, with the LP filter controller 306 resetting the low pass filter 304, as described in one or more embodiments above. Arrow 614 indicates where the signal processing location data 606 catches us to the real catheter location 602. The arrow 614 is positioned near *t*=5.5 seconds and is separated from the arrow 616 by a latency differential 612. In some embodiments, the latency differential 612 is greater than 0.5 seconds. Thus, the signal processing location data 606 provides improved responsiveness over the low pass filter location data 604 and attenuates cardiac motion with the same reliability and consistency as the low pass filter location data 604.

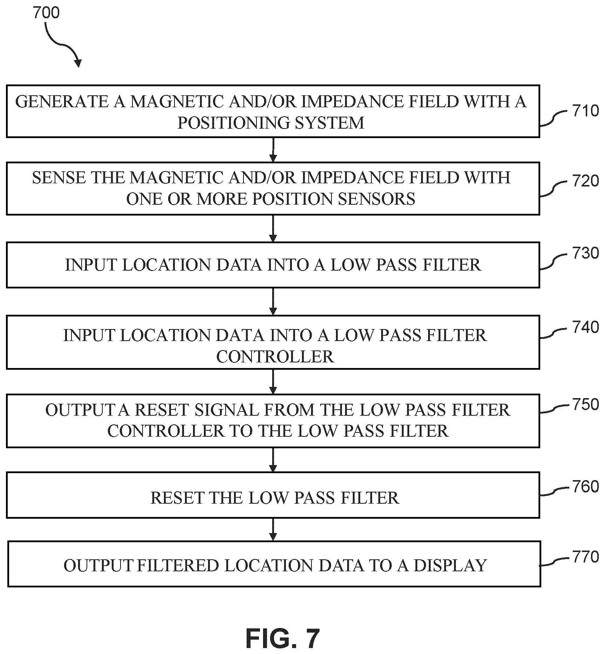

**FIG. 7** is a flow chart of a method 700 of displaying a visualized medical device, according to some embodiments. At step 710, the method 700 includes generating a magnetic field and/or an impedance field with a positioning system. In some embodiments, the magnetic field and/or the impedance field is generated by the magnetic field-based positioning system 118 and/or the impedance-based positioning system 116, respectively.

At step 720, the method 700 includes sensing the magnetic field and/or impedance field with one or more position sensors. The one or more position sensors include the one or more shaft electrodes 244, the one or more electrodes 256, and/or the magnetic position sensors, according to some embodiments. The sensed magnetic field and/or impedance field is communicated to the medical positioning system 120 to generate location data of the one or more position sensors.

At step 730, the method 700 includes inputting location data of the one or more position sensors into a low pass filter to attenuate oscillation of the location data from cardiac motion. In some embodiments, the low pass filter includes any or all features of the low pass filter 304 described above.

At step 740, the method 700 includes inputting the location data of the one or more position sensors into a low pass filter controller to determine whether a reset event has occurred. In some embodiments, the low pass filter controller includes any or all features of the LP filter controller 306 described above. The reset event includes a determination of medical device manipulation by a user, or in other words, a determination that motion of the one or more position sensors is attributable to deliberate catheter motion.

At step 750, the method 700 includes outputting a reset signal from the low pass filter controller. The reset signal is received by the low pass filter, according to some embodiments.

At step 760, the method 700 includes resetting the low pass filter. In some embodiments, resetting the low pass filter 304 includes modifying one or more input parameters of the low pass filter 304, including for example, a sample rate of the low pass filter, a cutoff frequency, a normalized passband frequency, a filter order, and/or a filter type. In some embodiments, resetting the low pass filter 304 includes inputting location data from a previous time interval into the low pass filter. In some embodiments, resetting the low pass filter 304 includes inputting a location estimate (e.g., the location estimate 414) into the low pass filter 304. Resetting the low pass filter 304 reduces latency of the filtered location data, according to some embodiments.

At step 770, the method 700 includes outputting filtered location data to a display. In some embodiments, the filtered location data is used to provide a real-time visualization model of the medical device.

Clause 1. A visualization system for a medical procedure, the system comprising: a medical device including one or more position sensors; a localization system to generate location data based on input received from the one or more position sensors; a signal processing system configured to receive the location data from the localization system, the signal processing system including: a low pass filter configured to generate filtered location data in response to the location data provided by the localization system, and a low pass filter controller configured to monitor the location data provided as an input to the low pass filter and the filtered location data to generate a reset output communicated to the low pass filter to reset the low pass filter; and a display providing real-time visualization of the medical device.

Clause 2. The visualization system of clause 1, wherein the reset of the low pass filter includes increasing a sample rate of the input location signals input into the low pass filter.

Clause 3. The visualization system of clause 1 or 2, wherein the reset of the low pass filter includes inputing a location estimate signal into the low pass filter.

Clause 4. The visualization system of clause 1, 2, or 3, wherein the reset output includes a reset metric, wherein one or more input parameters of the low pass filter are reconfigured based on the reset metric.

Clause 5. The visualization system of clause 3 or 4, wherein the low pass filter controller computes the location estimate signal of the one or more position sensors on the medical device, wherein the location estimate signal is computed by a moving statistical filter over a time interval.

Clause 6. The visualization system of clause 3, 4, or 5, wherein the reset metric is computed by determining a difference between the location estimate signal of the one or more position sensors on the medical device and the input location signals from the one or more position sensors.

Clause 7. The visualization system of clause 6, wherein the low pass filter controller determines whether a reset event has occurred by comparing the difference to a threshold, wherein if the difference is greater than the threshold the reset event has occurred and the reset output is generated.

Clause 8. A method of displaying a visualized medical device during a medical procedure, the method comprising: tracking one or more position sensors on a medical device with a positioning system; inputting location data of the one or more position sensors into a low pass filter to attenuate oscillation of the location data from cardiac motion; inputting the location data of the one or more position sensors into a low pass filter controller to determine whether a reset event occurred; outputting a reset signal from the low pass filter controller to the low pass filter; resetting the low pass filter; and outputting filtered location data to a display.

Clause 9. The method of clause 8, wherein resetting the low pass filter reduces latency of the filtered location data.

Clause 10. The method of clause 8 or 9, wherein resetting the low pass filter includes modifying one or more input parameters of the low pass filter, wherein the one or more input parameters include a sample rate of the low pass filter.

Clause 11. The method of clause 8, 9, or 10, wherein resetting the low pass filter includes inputting the location data from a previous time interval into the low pass filter.

Clause 12. The method of clause 8, 9, 10, or 11, further comprising: computing a location estimate of the one or more position sensors on the medical device with a moving statistical filter over a time interval computing a reset metric by determining a variance between the location estimate of the one or more position sensors on the medical device and the location data from the one or more position sensors, and comparing the variance to a threshold to determine whether the reset event has occurred.

Clause 13. The method of clause 12, wherein resetting the low pass filter includes inputting the location estimate of the one or more position sensors into the low pass filter.

Clause 14. A method of attenuating cardiac motion from medical device position data of a medical device, the method comprising: inputting location signals from one or more position sensors disposed on the medical device into a low pass filter to generate low pass filtered location data; inputting the location signals from the one or more position sensors into a low pass filter controller; determining whether motion of the one or more position sensors is attributable to manipulation of the medical device by a user; generating a reset output with the low pass filter controller when motion of the one or more position sensors is attributable to manipulation of the medical device by the user; and resetting the low pass filter.

Clause 15. The method of clause 14, wherein resetting the low pass filter includes modifying one or more input parameters of the low pass filter, wherein the one or more input parameters include a sample rate of the low pass filter.

Clause 16. The method of clause 15, wherein the one or more input parameters include a cutoff frequency, a normalized passband frequency, a filter order, and/or a filter type.

Clause 17. The method of clause 14, wherein resetting the low pass filter includes inputting the location signals from a previous time interval into the low pass filter.

Clause 18. The method of clause 14, wherein determining whether motion of the one or more position sensors is attributable to manipulation of the medical device by the user includes: computing an estimated cardiac motion parameter, computing a difference between the location signals from one or more position sensors and the estimated cardiac motion parameter, and comparing the difference to a threshold value.

Clause 19. The method of clause 18, wherein the estimated cardiac motion parameter includes one or more of a maximum distance displacement of the location signals, a slew rate of the location signals, and a slew rate of the low pass filtered location data.

Clause 20. The method of clause 19, wherein resetting the low pass filter includes inputting a location estimate of the one or more position sensors into the low pass filter, wherein the location estimate is computed via a moving statistical filter within a time interval of previous data.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1. A visualization system (100) for a medical procedure, the system comprising:
a medical device (102) including one or more position sensors;
a localization system (120) to generate location data (302) based on input received from the one or more position sensors;
a signal processing system (300, 400) configured to receive the location data (302) from the localization system (120), the signal processing system (300, 400) including:
a low pass filter (304) configured to generate filtered location data (309) in response to the location data (302) provided by the localization system (120), and
a low pass filter controller (306) configured to monitor the location data (302) provided as an input to the low pass filter (304) and the filtered location data (309) to generate a reset output (308) communicated to the low pass filter (304) to reset the low pass filter (304); and
a display (124) providing real-time visualization of the medical device.

2. The visualization system of claim 1, wherein the reset of the low pass filter (304) includes increasing a sample rate of the location data (302) input into the low pass filter (304).

3. The visualization system of claims 1 or 2, wherein the reset of the low pass filter (304) includes inputting a location estimate signal into the low pass filter (304).

4. The visualization system of claims 1, 2, or 3, wherein the reset output (308) includes a reset metric, wherein one or more input parameters of the low pass filter (304) are reconfigured based on the reset metric.

5. The visualization system of claims 3 or 4, wherein the low pass filter controller (306) computes the location estimate signal of the one or more position sensors on the medical device (102), wherein the location estimate signal is computed by a moving statistical filter over a time interval.

6. The visualization system of claims 4 or claim 5 as dependent on claim 4, wherein the reset metric is computed by determining a difference between the location estimate signal of the one or more position sensors (244, 256) on the medical device (102) and the input location signals (302) from the one or more position sensors.

7. The visualization system of claim 6, wherein the low pass filter controller (306) determines whether a reset event has occurred by comparing the difference to a threshold, wherein if the difference is greater than the threshold the reset event has occurred and the reset output (308) is generated.

8. A method of displaying a visualized medical device during a medical procedure, the method comprising:
tracking one or more position sensors on a medical device with a positioning system (step 710, 720);
inputting location data of the one or more position sensors into a low pass filter to attenuate oscillation of the location data from cardiac motion (step 730);
inputting the location data of the one or more position sensors into a low pass filter controller to determine whether a reset event occurred (step 740);
outputting a reset signal from the low pass filter controller to the low pass filter (step 750);
resetting the low pass filter (step 760); and
outputting filtered location data to a display (step 770).

9. The method of claim 8, wherein resetting the low pass filter reduces latency of the filtered location data.

10. The method of claims 8 or 9, wherein resetting the low pass filter includes modifying one or more input parameters of the low pass filter, wherein the one or more input parameters include a sample rate of the low pass filter.

11. The method of claims 8, 9, or 10, wherein resetting the low pass filter includes inputting the location data from a previous time interval into the low pass filter.

12. The method of claims 8, 9, 10, or 11, further comprising:
computing a location estimate of the one or more position sensors on the medical device with a moving statistical filter over a time interval
computing a reset metric by determining a variance between the location estimate of the one or more position sensors on the medical device and the location data from the one or more position sensors, and
comparing the variance to a threshold to determine whether the reset event has occurred.

13. The method of claim 12, wherein resetting the low pass filter includes inputting the location estimate of the one or more position sensors into the low pass filter.

14. The method of claims 8, 9, 10, 11, 12, or 13, wherein tracking one or more position sensors on a medical device with a positioning system (step 710, 720) further includes:
generate a magnetic and/or an impedance field with a positioning system (step 710);
sense the magnetic and/or the impedance field with one or more positioning sensors (step 720); and
utilize the sensed magnetic and/or impedance fields to generate location data.

15. The method of claims 8, 9, 10, 11, 12, or 13, wherein the reset event indicates that motion from the one or more position sensors is attributable to manipulation of the medical device.
